# EUROPEAN PATENT APPLICATION

(11) **EP 0 861 848 A1**
(43) Date of publication of application: **02.09.1998**
(21) Application number: 98103248.5
(22) Date of filing: 25.02.1998
(51) Int. Cl.: C07H 3/06, C08B 37/00

(54) **Glycolipid derivatives and use thereof**

(30) Priority: 28.02.1997 JP 45449/97
(71) Applicant: Chiba Flour Milling Co., Ltd., Chiba 261-0002 (JP)
(72) Inventor: Suzuki, Takanao, Chiba 261-0001 (JP); Yoshimura, Kiyoshi, Chiba 261-0012 (JP); Tsuchida, Eishun, Nerima-ku, Tokyo 177-0053 (JP); Takeoka, Shinji, Tokyo 158-0094 (JP); Sou, Keitaro, Tokyo 169-0051 (JP)
(74) Representative: von Kreisler, Alek, Dipl.-Chem.

(57) **Abstract**

Disclosed are (1) a glycolipid derivative in which a nitrogen atom-containing hydrophobic group is introduced into an anomer carbon atom of a reducing end group of an oligosaccharide having a saccharide polymerization degree of 5 to 30, and (2) a phospholipid vesicle stabilizer comprising a glycolipid derivative in which a nitrogen atom-containing hydrophobic group is introduced into an anomer carbon atom of a reducing end group of an oligosaccharide having a saccharide polymerization degree of 2 to 30.

## Description

### FIELD OF THE INVENTION

The present invention relates to a novel glycolipid derivative and a phospholipid vesicle stabilizer comprising the glycolipid derivative. More specifically, the present invention relates to a glycolipid derivative having a structure in which a hydrophobic group is introduced throgh a nitrogen atom into an anomer carbon atom of a reducing end group of an oligosaccharide and to a phospholipid vesicle stabilizer comprising the glycolipid derivative.

### BACKGROUND OF THE INVENTION

As well known, vesicles and fat emulsions have instability such as configurational alterations or leakage of contents caused by agglutination, fusion, freezing or drying, and many proposals for overcoming such instability have been submitted. For example, a method for solving such a problem has been attempted in which membranes of vesicles comprising phospholipids having unsaturated groups are polymerized to stabilize the membranes (for example, a review: H. Ringsdorf et al., Angew. Chem., Int. Engl., 27, 113 (1988)). However, the characteristics of the phospholipids before the polymerization, for example, gel-crystal phase transition, phase separation and membrane flow characteristics, are lost after the polymerization with loss of functions relating to these characteristics.

On the other hand, cholesterol derivatives containing β-aminogalactose have been used for agglutination control of vesicles (P-S. Wu, et al., Proc. Natl. Acad. Sci., USA, 78, 6211 (1981)). However, the effect of the derivatives is not so great as expected and it is suggested that the polymerization degree of saccharides is greatly concerned in the effect. Furthermore, hyalurone derivatives which exert stabilizing action on emulsion systems of vesicles are disclosed (Japanese Unexamined Patent Publication No. 3-47801). However, the hyalurone derivatives can not draw the stabilizing effect sufficiently because the derivatives have acyl groups non-selectively attached by ester linkages. The above-mentioned effect largely depends upon the positions and the number of the acyl groups introduced. Moreover, the viscosity becomes too high to put the derivatives to practical use in some cases.

The present inventors have disclosed that the introduction of oligosaccharide fatty acid esters previously developed by the inventors into bilayer membranes of vesicles is very effective for the dispersion stabilization of the vesicles (Japanese Unexamined Patent Publication No. 1-294701), and it is revealed that the fatty acid esters having longer saccharide chains have a more excellent effect. The above-mentioned oligosaccharide derivatives are non-selective in the positions of long-chain fatty acids introduced and random in the number of the acids introduced. However, one or two alkyl chains are successfully selectively introduced into an anomeric position of a reducing end group of the saccharide chain by lactonization of the anomeric position (Japanese Unexamined Patent Publication No. 5-317677) or by using trimethylsilylfluoromethanesulfonic acid as a catalyst after protection of hydroxy groups with acetyl groups and the like (Japanese Unexamined Patent Publication No. 5-294983), which is found to give a high dispersion stabilizing effect. However, these synthesis procedures are complicated and not practical. Accordingly, the advent of vesicle stabilizers which can be prepared by simpler procedures and exhibit high functionality has been desired.

On the other hand, N-glycosylcarboxamide derivatives of monosaccharides or maltose are proposed as antiinfection reinforcing agents (Japanese Examined Patent Publication No. 1-40036). However, it has not been known at all that the derivatives have the action of stabilizing vesicles. Further, the laid-open patent (Japanese Unexamined Patent Publication No. 58-188891) corresponding thereto refers to oligosaccharides, such as trisaccharides and tetrasaccharides. However, specific examples thereof are limited to monosaccharides and disaccharides.

The saccharide derivatives disclosed in Japanese Unexamined Patent Publication Nos. 5-317677 and 5-294983 described above fairly contribute to the stabilization of vesicles. However, the termini of the oligosaccharides must be lactonized or the hydroxyl groups must be protected. Further, the presence of unstable intermediates necessitates minute care, labor and time. The above-mentioned conventional procedures are complicated in synthesis, so that they have been desired to be simplified in purification operations and improved in yield. The present inventors have intensively studied for developing glycolipid derivatives of commercial importance in which hydrophobic groups can be directly introduced into terminal anomeric positions of oligosaccharides, whose yield is high, and whose purification process is simple.

### SUMMARY OF THE INVENTION

In view of the above situation, the present inventors conducted intensive investigations. As a result, the present inventors have discovered that hydrophobic groups can be selectively introduced into terminal anomeric positions of oligosaccharides by high reactivity of reducing termini of the oligosaccharides and amino groups, according to a simple method, and that the resulting products have as phospholipid vesicle stabilizers a stabilizing effect equal to or greater than that of conventional stabilizers, thus completing the present invention.

That is, the present invention provides:
(1) A glycolipid derivatives in which a nitrogen atom-containing hydrophobic group is introduced into an anomer carbon atom of a reducing end group of an oligosaccharide having a saccharide polymerization degree of 5 to 30;
(2) The glycolipid derivative described in (1), which is represented by general formula (I): 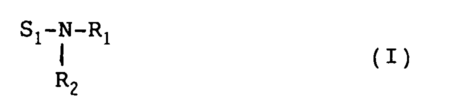 wherein S₁ represents an oligosaccharide having a saccharide polymerization degree of 5 to 30; R₁ represents an aliphatic hydrocarbon group having 12 to 22 carbon atoms; and R₂ represents hydrogen, an aliphatic hydrocarbon group having 1 to 22 carbon atoms or an acyl group having 2 to 22 carbon atoms;
(3) The glycolipid derivative described in (1) or (2), in which the oligosaccharide is a maltooligosaccharide having a glucose unit number of 5 to 30.
(4) The glycolipid derivative described in (1), (2) or (3), in which the oligosaccharide is a maltooligosaccharide having a glucose unit number of 5 to 30 and the nitrogen atom-containing hydrophobic group is an alkyl group having 12 to 22 carbon atoms;
(5) A phospholipid vesicle stabilizer comprising a glycolipid derivative in which a nitrogen atom-containing hydrophobic group is introduced into an anomer carbon atom of a reducing end group of an oligosaccharide having a saccharide polymerization degree of 2 to 30;
(6) The phospholipid vesicle stabilizer described in (5), which is represented by general formula (II): 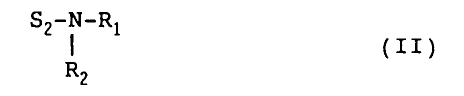 wherein S₂ represents an oligosaccharide having a saccharide polymerization degree of 2 to 30; R₁ represents an aliphatic hydrocarbon group having 12 to 22 carbon atoms; and R₂ represents hydrogen, an aliphatic hydrocarbon group having 1 to 22 carbon atoms or an acyl group having 2 to 22 carbon atoms;
(7) The phospholipid vesicle stabilizer described in (5) or (6), in which the oligosaccharide in the glycolipid derivative is a maltooligosaccharide having a glucose unit number of 2 to 30; and
(8) The phospholipid vesicle stabilizer described in (5), (6) or (7), in which the oligosaccharide in the glycolipid derivative is a maltooligosaccharide having a glucose unit number of 2 to 30 and the nitrogen atom-containing hydrophobic group is an alkyl group having 12 to 22 carbon atoms;

### DESCRIPTION OF THE PREFERRED EMBODIMENTS

The present invention is illustrated in detail below. Saccharides used for preparing the glycolipid derivatives of the present invention are ones having reducing terminal groups composed of aldoses or ketoses, and may be branched or straight-chain oligosaccharides having a saccharide polymerization degree of 2 to 30. They may be either naturally occurred or synthetic saccharides. Specifically, they are saccharides in which one or more kinds of glucose, fructose, xylose, galactose, mannose and glucosamine are linked by α- or β-linkages. Any saccharides can be used as long as they have reducing terminal groups composed of aldoses or ketoses. Examples thereof include maltooligosaccharides, laminarioligosaccharides, cellooligosaccharides, isomaltooligosaccharides, gentiooligosaccharides, nigerooligosaccharides, lactooligosaccharides, melioligosaccharides, inulooligosaccharides, degradation products of starch, degradation products of pullulan, degradation products of cellulose, degradation products of other polysaccharides, and synthesized saccharides. Mixtures of the above-mentioned oligosaccharides can also be used. Of these, maltooligosaccharides having a glucose unit number of 5 to 30, preferably of 5 to 10, (for example, maltopentaose, maltohexaose, maltoheptaose, maltooctaose, maltononaose, maltodecaose or mixtures thereof) are preferred. The glycolipid derivatives used as the phospholipid vesicle stabilizers in the present invention, oligosaccharides having a saccharide polymerization degree of 2 to 30 are used, and the others are the same as described above. In particular, maltooligosaccharides having a glucose unit number of 2 to 30, preferably of 5 to 10, are preferred.

From the importance of the adjustment of hydrophile-hydrophobe balance and steric structures of molecules, the nitrogen atom-containing hydrophobic groups used for preparing the glycolipid derivatives of the present invention are primary amines containing aliphatic hydrocarbon groups each having 12 to 22 carbon atoms, preferably 16 to 18 carbon atoms, for example, alkyl, alkenyl or alkynyl, or secondary amines containing aliphatic hydrocarbon groups each having 12 to 22 carbon atoms, preferably 16 to 18 carbon atoms, and aliphatic hydrocarbon groups each having 1 to 22 carbon atoms, for example, alkyl, alkenyl or alkynyl. When an unsaturated group(s) is contained, the number of the unsaturated group(s) is preferably from 1 to 4. Examples of the primary amines include dodecylamine, tridecylamine, tetradecylamine, pentadecylamine, hexadecylamine, heptadecylamine, octadecylamine, docosylamine and oleylamine, each also including a branched chain. Further, amines such as branched isopreonids can also be used. The secondary amines include N-methyl-dodecylamine, N-methyl-tetradecylamine, N-methyl-hexadecylamine, N-ethyl-dodecylamine, N-ethyl-tetradecyl-amine, N-ethyl-hexadecylamine, N-propyl-dodecylamine, N-propyl-tetradecylamine, N-propyl-hexadecylamine and dioleylamine, each also including a branched chain.

The glycolipids with which the primary amines have been allowed to react can be further allowed to react with acid anhydrides or acid chlorides having 2 to 22 carbon atoms at the nitrogen atoms. Fatty acids used in the acid anhydrides or the acid chlorides include acetic acid, propionic acid, butyric acid, valeric acid, isovaleric acid, caproic acid, enanthic acid, caprylic acid, pelargonic acid, capric acid, undecanoic acid, lauric acid, tridecanoic acid, myristic acid, pentadecanoic acid, palmitic acid, margaric acid, stearic acid, nonadecanoic acid, arachic acid, behenic acid, oleic acid, linoleic acid, linolenic acid and arachidonic acid.

The nitrogen atom-containing hydrophobic groups are introduced into the terminal anomer carbon atoms of saccharide chains by allowing the above-mentioned oligosaccharides having reducing termini to react with the above-mentioned amines in reaction solvents, preferably in an atmosphere of nitrogen at about 20°C to about 80°C for 1 hour to 6 hours. The reaction solvents which can be used include lower alcohols such as methanol, ethanol, propanol, isopropanol, butanol, tert-butanol and isobutanol, ethers such as diisopropyl ether and diethyl ether, lower ketones, esters such as ethyl acetate and butyl acetate, dimethylformamide, formamide, chloroform, dichloromethane, dioxane, tetrahydrofuran, benzene, toluene and hexane. In the Lockhoff method of introducing alkyl chains into the terminal anomeric positions of monosaccharides and disaccharides (O. Lockhoff, Angew. Chem. Int. Ed. Engl., 30, 1611 (1991)), the reaction within 1 hour in the atmosphere is described. However, when the hydrophobic groups are introduced into the oligosaccharides having a saccharide polymerization degree of 5 or more, starting materials turn brown or black in the atmosphere during reaction. It is therefore preferred that the reaction is carried out in an atmosphere of nitrogen.

The acid anhydrides or the acid chlorides are introduced by allowing them to react in solvents or in water-organic solvents at 0°C to 80°C. The reaction solvents include dioxane, dimethylformamide, dimethyl sulfoxide and N-methylpyrrolidinone, and the organic solvents mixed with water include benzene, toluene, xylene, hexane, methanol, ethanol, butanol and acetone.

For the purification of products, unreacted hydrophobic groups containing amino groups and fatty acids are removed by extraction operations with solvents in which these substances are highly soluble. Such solvents include hexane, chloroform, dichloromethane, benzene, toluene and xylene. Unreacted saccharides are extracted with aqueous solutions or can be fractionated by use of dichloromethane, methanol or water as a mobile phase and silica gel as a stationary phase. The products are confirmed by nuclear magnetic resonance spectroscopy, infrared spectroscopy, mass spectrometry and elemental analysis.

The purity of the glycolipids thus obtained are examined by thin layer chromatography and high performance liquid chromatography.

Examples of the glycolipid derivatives of the present invention are shown by structural formulas below. 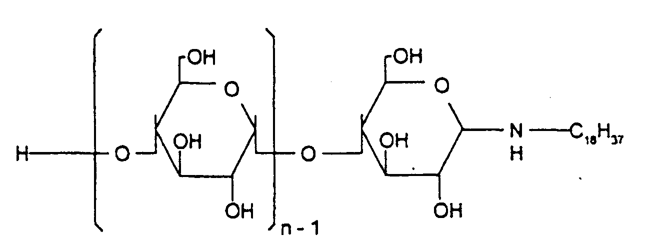 or

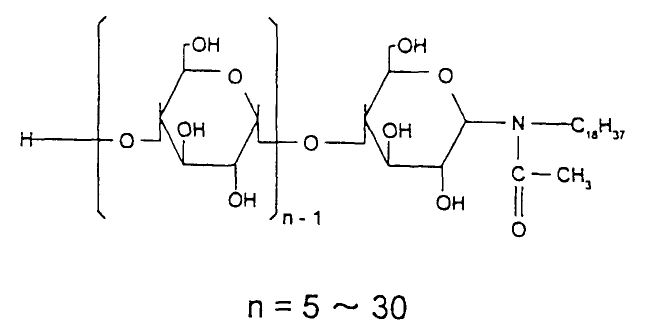

In the phospholipid vesicle stabilizers comprising the glycolipid derivatives of the present invention, the glycolipid derivatives in which the nitrogen atom-containing hydrophobic groups are introduced into the anomer carbon atoms of the reducing termini of the oligosaccharides having a saccharide polymerization degree of 2 to 30, particularly the glycolipid derivatives represented by general formula (II), are used. 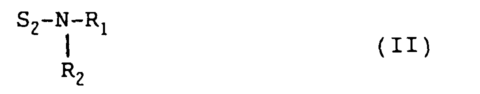 wherein S₂ represents an oligosaccharide having a saccharide polymerization degree of 2 to 30; R₁ represents an aliphatic hydrocarbon group having 12 to 22 carbon atoms; and R₂ represents hydrogen, an aliphatic hydrocarbon group having 1 to 22 carbon atoms or an acyl group having 2 to 22 carbon atoms.

Stabilization of the vesicles by use of the glycolipid derivatives of the present invention can be achieved in the following manner.

The phospholipid vesicle stabilizers of the present invention are added in an amount of 2 mol% to 50 mol%, preferably in an amount of 5 mol% to 10 mol%, to mixed lipids comprising phospholipids as main components, sterols such as cholesterol and cholestanol, or fatty acids and negatively charged phospholipids such as phosphatidic acid and phosphatidyl glycerol to prepare hydrates. Vesicle dispersions with a diameter of 30 to 200 nm are prepared from said hydrates by known methods using ultrasonic agitators, microfluidizers or extruders.

Further, it is also possible to introduce the phospholipid vesicle stabilizers of the present invention only into outer surfaces of the vesicles by adding aqueous solutions of the stabilizers to dispersed solutions of the vesicles prepared beforehand. The phospholipid vesicle stabilizers are added in an amount of 1 mol% to 30 mol%, preferably 3 mol% to 10 mol%, to the dispersed solutions of the vesicles and allowed to stand at 5°C to 80°C for some time, preferably for 30 minutes or more, thereby introducing the vesicle stabilizers into the vesicles.

The particle size of the vesicles in the vesicle dispersions can be confirmed by use of a particle size distribution measuring device or by observation of samples treated by negative staining or freeze-fructure under a transmission electron microscope.

The above-mentioned dispersions of the vesicles are subjected to gel filtration or ultracentrifugation to remove the stabilizers which have not been introduced into the vesicles to the outsides of the systems, followed by quantitative determination of saccharides in respective fractions by the phenol-sulfuric acid method, thereby being able to confirm that 90% or more of the phospholipid vesicle stabilizers added have been introduced into the phospholipid vesicles. The dispersion stability can be readily estimated by measuring the variation of absorbance (turbidity) at 300 nm to 800 nm with the lapse of time with a visible-light spectrophotometer, for the dispersions from which the free phospholipid vesicle stabilizers have been removed by gel filtration or ultracentrifugation, and which are allowed to stand at room temperature. When the dispersions are not stabilized, agglutination or fusion of the vesicles occurs to increase the turbidity.

Evaluation methods of the dispersion stability include a method of measuring the viscosity of the vesicle dispersions with a cone plate type rotational viscometer and a microscopic method by use of an optical microscope or an electron microscope, as well as the above-mentioned turbidity measuring method.

The glycolipid derivatives of the present invention are homogeneously introduced into phospholipid bilayer membranes of the vesicles to function as the stabilizers for the phospholipid vesicles (hereinafter referred to as "vesicles"), because of their high affinity for the phospholipids. In addition, the glycolipid derivatives introduced into the vesicles retain the state where chains of the saccharides are exposed to the aqueous phase, and can be homogeneously dispersed on the membranes of the vesicles.

The glycolipid derivatives of the present invention used as the phospholipid vesicle stabilizers can be appropriately used according to the purposes of use such as dispersion stabilization of proteins, label formation for recognition by specific antibodies and inhibition of phagocytosis in vivo, as well as inhibition of agglutination of the vesicles, prevention of membrane fusion and inhibition of leakage of contents in the vesicles.

When the vesicles contain the proteins, they are desirably stored in the low-temperature, frozen or dried state. The vesicles modified with the glycolipid derivatives of the present invention do not agglutinate and fuse even in repeated operations of freezing and thawing, and can be stored as frozen vesicles. Moreover, it is also possible to store them as dried vesicles obtained by lyophilization or spray drying.

When carriers in which aqueous phases of the vesicles contain functional polymers are administered in vivo, longer retention time in blood results in higher effectiveness. However, the vesicles agglutinate by interaction with proteins or calcium ions existing in plasma, and are sometimes excluded from blood by uptake in reticuloendothelial systems and phagocytes for a short period of time. However, the retention time of the vesicles in blood can be controlled by surface modification of the vesicles with the glycolipid derivatives of the present invention.

Further, the glycolipid derivatives of the present invention having unsaturated bonds in their hydrophobic moieties are most suitable for using them in combination with polymerizable phospholipids, for example, for applying them onto surfaces of incubators, followed by copolymerization to convert the surfaces of the incubators to hydrophilic and cytophilic surfaces, and give to the surfaces of the incubators a great culture effect which has been never seen.

In the phospholipid vesicle stabilizers of the present invention, the reaction sites are restricted by the method of combining the reducing termini of the oligosaccharides having a saccharide polymerization degree of 2 to 30 with amines having hydrocarbon chains, and then, binding acyl groups or hydrocarbon chains to the N-positions. Accordingly, no protective groups are required in the synthesis of the glycolipid derivatives, which makes it possible to simply synthesize them, and it is unnecessary to use HPLC in the purification process, because of utilization of the difference in solubility from the raw materials. The glycolipid derivatives can therefore be supplied in high yields in large amounts at low cost. The phospholipid vesicle stabilizers of the present invention comprising these glycolipid derivatives are used as stabilizers for vesicles and fat emulsions in the fields of cosmetics, drugs and industry. This enables dispersion stabilization to ions and long-term stable storage, as well as dispersion stabilization such as inhibition of agglutination or fusion of the vesicles or the fat emulsions themselves, and it becomes possible to use the stabilizers of the present invention as stabilizers to refusion after lyophilization and pulverization, which is excellent in economy and performance.

The present invention will be described in more detail with reference to examples below, but is not limited thereby.

### EXAMPLE I (Production Examples)

### EXAMPLE I-1

A maltopentaose (400 mg) and octadecylamine (262 mg) were dissolved in 10 ml of dimethylformamide (DMF), and allowed to react in an atmosphere of nitrogen at 80°C for 5 hours. The progress of the reaction was confirmed by disappearance of the spot of the maltopentaose (Rf = 0.05) and appearance of a new spot (Rf = 0.53) using thin layer chromatography (Silica Gel 60, Merck Co., developing solvent: chloroform/methanol/water = 5/4/1, detection: sulfuric acid). The reaction was stopped at the time when the single spot of Rf = 0.53 appeared. The reaction solution was concentrated under reduced pressure and washed with hexane, followed by drying to obtain 418 mg of a light yellow powder. From appearance of a peak of amine-derived N-H at 1600 cm⁻¹ in IR spectrum and a peak of a secondary amine in ¹³C-NMR (solvent: heavy DMSO), it was confirmed that octadecylamine was bound to the anomer carbon atom of the maltopentaose.

### EXAMPLE I-2

A maltooligosaccharide having an average saccharide polymerization degree of 11.3 (500 mg) and octadecylamine (160 mg) were dissolved in 10 ml of dimethylformamide (DMF), and allowed to react in an atmosphere of nitrogen at 80°C for 5 hours. The progress of the reaction was confirmed by disappearance of the spot of the maltooligosaccharide (Rf = 0.03) and appearance of a new spot (Rf = 0.32) using thin layer chromatography (Silica Gel 60, Merck Co., developing solvent: chloroform/methanol/water = 5/4/1, detection: sulfuric acid). The reaction was stopped at the time when the single spot of Rf = 0.32 appeared. The reaction solution was concentrated under reduced pressure and washed with hexane, followed by drying to obtain 460 mg of a light yellow powder. From appearance of a peak of amine-derived N-H at 1600 cm⁻¹ in IR spectrum and a peak of a secondary amine in ¹³C-NMR (solvent: heavy DMSO), it was confirmed that octadecylamine was bound to the anomer carbon atom of the maltooligosaccharide.

### EXAMPLE I-3

A maltooligosaccharide having an average saccharide polymerization degree of 20.4 (500 mg) and octadecylamine (80 mg) were dissolved in 10 ml of dimethylformamide (DMF) and allowed to react in an atmosphere of nitrogen at 80°C for 5 hours. The progress of the reaction was confirmed by disappearance of the spot of the maltooligosaccharide (Rf = 0.01) and appearance of a new spot (Rf = 0.15) using thin layer chromatography (Silica Gel 60, Merck Co., developing solvent: chloroform/methanol/water = 5/4/1, detection: sulfuric acid). The reaction was stopped at the time when the single spot of Rf = 0.15 appeared. The reaction solution was concentrated under reduced pressure and washed with hexane, followed by drying to obtain 430 mg of a light yellow powder. From appearance of a peak of amine-derived N-H at 1600 cm⁻¹ in IR spectrum and a peak of a secondary amine in ¹³C-NMR (solvent: heavy DMSO), it was confirmed that octadecylamine was bound to the anomer carbon atom of the maltooligosaccharide.

### EXAMPLE I-4

The glycolipid derivative (200 mg) obtained in Example I-2 was dissolved in 5 ml of dimethylformamide (DMF), and palmitoyl chloride (60 mg) was added dropwise thereto at 20°C, followed by reaction for 3 hours. The reaction product was reprecipitated with acetone, washed with water, and then dried to obtain 160 mg of a light yellow powder. From appearance of a peak of amide-derived C=O at 1640 cm⁻¹ in IR spectrum and a peak of C=O in ¹³C-NMR (solvent: heavy DMSO), it was confirmed that palmitic acid was bound to the secondary amine.

### EXAMPLE I-5

A maltooligosaccharide having a saccharide polymerization degree of 7 (500 mg) and dodecylamine (170 mg) were dissolved in 10 ml of dimethylformamide (DMF) and allowed to react in an atmosphere of nitrogen at 80°C for 5 hours. The progress of the reaction was confirmed by disappearance of the spot of the maltooligosaccharide (Rf = 0.04) and appearance of a new spot (Rf = 0.30) using thin layer chromatography (Silica Gel 60, Merck Co., developing solvent: chloroform/methanol/water = 5/4/1, detection: sulfuric acid). The reaction was stopped at the time when the single spot of Rf = 0.30 appeared. The reaction solution was concentrated under reduced pressure and washed with hexane, followed by drying to obtain 420 mg of a light yellow powder. From appearance of a peak of amine-derived N-H at 1600 cm⁻¹ in IR spectrum and a peak of a secondary amine in ¹³C-NMR (solvent: heavy DMSO), it was confirmed that the amine was bound to the anomer carbon atom of the maltooligosaccharide. The resulting glycolipid derivative (200 mg) was dissolved in 5 ml of dimethylformamide (DMF), and acetic anhydride (10 mg) was added dropwise thereto at 20°C, followed by reaction for 3 hours. The reaction product was reprecipitated with acetone, washed with water, and then dried to obtain 140 mg of a light yellow powder. From appearance of a peak of amide-derived C=O at 1640 cm⁻¹ in IR spectrum and a peak of C=O in ¹³C-NMR (solvent: heavy DMSO), it was confirmed that acetic acid was bound to the secondary amine.

### EXAMPLE I-6

The glycolipid derivative (200 mg) obtained in Example I-3 was dissolved in 5 ml of dimethylformamide (DMF), and octadecadienoyl chloride (36 mg) was added dropwise thereto at 20°C, followed by reaction for 3 hours. The reaction product was reprecipitated with acetone, washed with water, and then dried to obtain 140 mg of a light yellow powder. From appearance of a peak of amide-derived C=O at 1640 cm⁻¹ in IR spectrum and a peak of C=O in ¹³C-NMR (solvent: heavy DMSO), it was confirmed that octadecadienoic acid was bound to the secondary amine.

### EXAMPLE I-7

A maltooligosaccharide having an average saccharide polymerization degree of 28.2 (500 mg) and docosylamine (70 mg) were dissolved in 10 ml of dimethylformamide (DMF), and allowed to react in an atmosphere of nitrogen at 80°C for 5 hours. The progress of the reaction was confirmed by disappearance of the spot of the maltooligosaccharide (Rf = 0.01) and appearance of a new spot (Rf = 0.17) using thin layer chromatography (Silica Gel 60, Merck Co., developing solvent: chloroform/methanol/water = 5/4/1, detection: sulfuric acid). The reaction was stopped at the time when the single spot of Rf = 0.17 appeared. The reaction solution was concentrated under reduced pressure and washed with hexane, followed by drying to obtain 430 mg of a light yellow powder. From appearance of a peak of amine-derived N-H at 1600 cm⁻¹ in IR spectrum and a peak of a secondary amine in ¹³C-NMR (solvent: heavy DMSO), it was confirmed that the amine was bound to the anomer carbon atom of the maltooligosaccharide. The resulting glycolipid derivative (200 mg) was dissolved in 5 ml of dimethylformamide (DMF), and p-nitrophenyl docosanoate (100 mg) was added dropwise thereto at 20°C, followed by reaction for 3 hours. The reaction product was reprecipitated with acetone, washed with water, and then dried to obtain 130 mg of a light yellow powder. From appearance of a peak of amide-derived C=O at 1640 cm⁻¹ in IR spectrum and a peak of C=O in ¹³C-NMR (solvent: heavy DMSO), it was confirmed that docosanoic acid was bound to the secondary amine.

### EXAMPLE II (Stabilization)

### EXAMPLE II-1

Dipalmitoyl phosphatidylcholine (DPPC) (50 mg) was dissolved in chloroform and the resulting solution was dried under reduced pressure to form a thin membrane. Distilled water (10 ml) was added thereto and the mixture was subjected to probe type ultrasonic agitation to obtain a milky transparent dispersion of vesicles (particle size: 35 nm). Each of the glycolipid derivatives obtained in Examples I-1 and I-2 and an aqueous solution of maltosyl octadecylamine (3.2 mM, 2 ml) was added to 2 ml of the dispersion, and each mixture was allowed to stand at 50°C for 1 hour. Thus, the glycolipid derivatives could be introduced into surfaces of the DPPC vesicles. The unintroduced glycolipid derivatives in supernatant solutions were removed by centrifugation (30,000 g, 60 minutes) and quantitatively determined by the phenol-sulfuric acid method to calculate introducing rates (Table 1).

The vesicles recovered as precipitates were redispersed into hot water of 50°C, and the resulting dispersions were allowed to stand at 25°C for 24 hours. Then, changes in turbidity were determined from changes in optical density (λ = 500 nm) measured on a visible light spectrophotometer. The unmodified vesicles exhibited the largest change in optical density (ΔO.D.) and the change decreased in the order of the maltose derivative, the oligosaccharide derivative (average polymerization degree: 11.3) and the maltopentaose derivative (Table 1).

**Table 1**

| (Introducing Rates of Glycolipid Derivatives and Changes in Turbidity) | | |
|---|---|---|
| Glycolipid Derivative | Introducing Rate (%) | ΔO.D. |
| Unmodified | - | 0.25 |
| Maltose Derivative | 93 | 0.10 |
| Production Example 1 | 91 | 0.01 |
| Production Example 2 | 65 | 0.05 |

### EXAMPLE II-2

Dipalmitoyl phosphatidylcholine (DPPC) (200 mg), cholesterol (106 mg), dipalmitoyl phosphatidylglycerol (DPPG) (58 mg) and the glycolipid derivative obtained in Example I-3 (124 mg) were dissolved in a mixed solvent (chloroform/methanol), and the resulting solution was dried under reduced pressure to form a thin membrane. The membrane was hydrated with 5 ml of a buffer solution (HEPES 20 mM, NaCl 140 mM), and vesicles adjusted to 200 nm in particle size by extrusion (final filter pore size: 0.2 µm) was prepared. The free glycolipid derivative was removed by centrifugation (30,000 g, 60 minutes). The introducing rate of the glycolipid derivative into the vesicles was found to be 95%. As a comparative example, vesicles were similarly prepared from dipalmitoyl phosphatidylcholine (DPPC) (200 mg), cholesterol (106 mg) and dipalmitoyl phosphatidylglycerol (DPPG) (58 mg).

The vesicles recovered as a precipitate were redispersed in a buffer solution (HEPES 20 mM, NaCl 140 mM) so as to give a concentration of 10% by weight, and distilled water or a 4-mM aqueous solution of Ca²⁺ in a volume equal to that of the dispersion was added thereto. Then, the solution viscosity was measured with a cone plate type rotational viscometer. In the system into which the glycolipid derivative was not introduced, addition of the aqueous solution of Ca²⁺ caused agglutination of the vesicles, and particularly, a remarkable rise in solution viscosity was observed in the shear rate region. However, in the system into which the glycolipid derivative was introduced, no increase in solution viscosity due to addition of the aqueous solution of Ca²⁺ was observed.

### EXAMPLE II-3

Distearoyl phosphatidylcholine (DSPC) (100 mg), cholesterol (50 mg), dipalmitoyl phosphatidylglycerol (DPPG) (28 mg), the glycolipid derivative obtained in Example I-4 (61 mg) were dissolved in a mixed solvent (chloroform/methanol) and the resulting solution was dried under reduced pressure to form a thin membrane. The membrane was hydrated with 5 ml of a buffer solution (HEPES 20 mM, NaCl 140 mM) and vesicles adjusted to 200 nm in particle size were prepared by extrusion (final filter pore size: 0.2 µm). The free glycolipid derivative was removed by centrifugation (30,000 g, 60 minutes). The introducing rate of the glycolipid derivative into the vesicles was 96%. As an comparative example, vesicles were similarly prepared from distearoyl phosphatidylcholine (DSPC) (100 mg), cholesterol (50 mg), and dipalmitoyl phosphatidylglycerol (DPPG) (28 mg).

The vesicles recovered as a precipitate were redispersed into a buffer solution (HEPES 20 mM, NaCl 140 mM). The resulting dispersion was freezed at -40°C and melted at 25°C. After this operation was repeated four times, the particle size of the vesicles was measured with a particle size distribution measuring device. In the system into which the glycolipid derivative was not introduced, a new peak was observed at 500 nm by agglutination induction of the vesicles and the intensity thereof increased with the number of repetitions of the operation. In the system into which the glycolipid derivative was introduced, such a change in particle size was not observed.

### EXAMPLE II-4

Dimyristoyl phosphatidylcholine (DMPC) (100 mg), cholesterol (50 mg), dipalmitoyl phosphatidylglycerol (DPPG) (28 mg) and the glycolipid obtained in Example I-7 (61 mg) were dissolved in a mixed solvent (chloroform/methanol), and the resulting solution was dried under reduced pressure to form a thin membrane. The membrane was hydrated 5 ml of an aqueous solution of carboxyfluorescein (CF) and the average particle size thereof was adjusted to 200 nm with a microfluidizer. CF which was not internally capsulated and the free glycolipid derivative were removed by gel filtration. As a comparative example, vesicles were similarly prepared from dimyristoyl phosphatidylcholine (DMPC) (100 mg), cholesterol (50 mg), and dipalmitoyl phosphatidylglycerol (DPPG) (28 mg).

The dispersion was lyophilized, and redispersed into distilled water. Then, the leakage rate of internally capsulated CF was measured. In the system into which the glycolipid derivative was not introduced, the leakage rate amounted to 50%, whereas in the system into which the glycolipid derivative was introduced, it was suppressed to 10%.

### EXAMPLE II-5

Dioctadecadienoyl phosphatidylcholine (100 mg) and the glycolipid derivative obtained in Example I-6 (23 mg) were dissolved in a mixed solvent (chloroform/methanol), and the resulting solution was dried under reduced pressure to form a thin membrane. The membrane was hydrated with 10 ml of distilled water, and the particle size thereof was adjusted to 200 nm by extrusion (final filter pore size: 0.2 µm). After removal of the free glycolipid derivative by ultracentrifugation, copolymerization was conducted under ice cooling by ultraviolet irradiation (for 30 minutes) with a low pressure mercury lamp. After spray drying, the resulting product was redispersed into distilled water, and the particle size thereof was measured with a particle diameter distribution measuring device. As a result, the particle size of 200 nm was completely kept.

Vesicles of dioctadecadienoyl phosphatidylcholine into which the glycolipid was not introduced were similarly polymerized, and the resulting product was spray dried to obtain a powder. The powder thus obtained was poorly redispersed into distilled water, and a number of aggregates having a size of 400 nm or more were observed.

The glycolipid derivatives of the present invention are useful for giving stability to the phospholipid vesicles. Use of the glycolipid derivatives as modifiers for the vesicles makes enables dispersion stabilization to ions, stabilization to lyophilization and pulverization which makes long-term stable storage possible, or application to cosmetics such as beauty lotion and skin lotion, as well as dispersion stabilization such as inhibition of agglutination and fusion of the vesicles themselves. The glycolipid derivatives can be simply synthesized without no need for protective groups because of the restricted reaction sites, and obtained in high yields without use of HPLC in the purification process. Accordingly, the phospholipid vesicle stabilizers of the glycolipid derivatives are excellent in economy and performance, and expected to have many applications.

## Claims

1. A glycolipid derivative in which a nitrogen atom-containing hydrophobic group is introduced into an anomer carbon atom of a reducing end group of an oligosaccharide having a saccharide polymerization degree of 5 to 30.

2. The glycolipid derivative according to claim 1, which is represented by general formula (I): 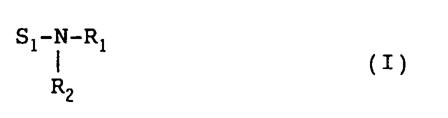 wherein S₁ represents an oligosaccharide having a saccharide polymerization degree of 5 to 30; R₁ represents an aliphatic hydrocarbon group having 12 to 22 carbon atoms; and R₂ represents hydrogen, an aliphatic hydrocarbon group having 1 to 22 carbon atoms or an acyl group having 2 to 22 carbon atoms.

3. The glycolipid derivative according to claim 1 or 2, in which the oligosaccharide is a maltooligosaccharide having a glucose unit number of 5 to 30.

4. The glycolipid derivative according to claim 1, 2 or 3, in which the oligosaccharide is a maltooligosaccharide having a glucose unit number of 5 to 30 and the nitrogen atom-containing hydrophobic group is an alkyl group having 12 to 22 carbon atoms;

5. A phospholipid vesicle stabilizer comprising a glycolipid derivative in which a nitrogen atom-containing hydrophobic group is introduced into an anomer carbon atom of a reducing end group of an oligosaccharide having a saccharide polymerization degree of 2 to 30.

6. The phospholipid vesicle stabilizer according to claim 5, which is represented by general formula (II): 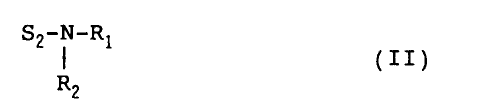 wherein S₂ represents an oligosaccharide having a saccharide polymerization degree of 2 to 30; R₁ represents an aliphatic hydrocarbon group having 12 to 22 carbon atoms; and R₂ represents hydrogen, an aliphatic hydrocarbon group having 1 to 22 carbon atoms or an acyl group having 2 to 22 carbon atoms.

7. The phospholipid vesicle stabilizer according to claim 5 or 6, in which the oligosaccharide in the glycolipid derivative is a maltooligosaccharide having a glucose unit number of 2 to 30.

8. The phospholipid vesicle stabilizer according to claim 5, 6 or 7, in which the oligosaccharide in the glycolipid derivative is a maltooligosaccharide having a glucose unit number of 2 to 30 and the nitrogen atom-containing hydrophobic group is an alkyl group having 12 to 22 carbon atoms.
